# EUROPEAN PATENT APPLICATION

(11) **EP 3 424 521 A1**
(43) Date of publication of application: **09.01.2019**
(21) Application number: 16891976.9
(22) Date of filing: 01.03.2016
(51) Int. Cl.: A61K 38/26, A61K 47/02, A61K 47/10, A61P 3/10

(54) **PHARMACEUTICAL COMPOSITION AND MANUFACTURING METHOD THEREOF**

(71) Applicant: Hybio Pharmaceutical Co., Ltd, Shenzhen, Guangdong 518057 (CN)
(72) Inventor: DAI, Ronghuan, Shenzhen Guangdong 518057 (CN); ZHANG, Lei, Shenzhen Guangdong 518057 (CN); QIN, Liangzheng, Shenzhen Guangdong 518057 (CN); TAO, Anjin, Shenzhen Guangdong 518057 (CN); YUAN, Jiancheng, Shenzhen Guangdong 518057 (CN)
(74) Representative: HGF Limited
(86) International application number: PCT/CN2016/075172
(87) International publication number: WO 2017/147783

(57) **Abstract**

The present invention relates to the field of polypeptides, and particularly, provides a pharmaceutical composition and a manufacturing method thereof. The pharmaceutical composition comprises a Liraglutide, and the manufacturing method of the pharmaceutical composition comprises: mixing, in a solvent, the Liraglutide and an adjuvant, stirring the resultant mixture at 500-1100 rpm until homogeneous, and adjusting the pH value to 7.5-9.5. Various manufacturing process parameters can influence the stability of Liraglutide and may cause significant changes in oligomerization, single maximal impurity, and total impurity. The infrared spectra show an amide band I (at about 1645 nm⁻¹), indicating the presence of an α-helix structure, with strong absorption and a basically consistent peak shape. The present invention controls, by examination of the secondary structure of a polypeptide, parameter screening of a pharmaceutical preparation process.

## Description

### FIELD

The present invention relates to the field of polypeptides, and in particular to a pharmaceutical composition and a manufacturing method thereof.

### BACKGROUND

Glucagon-like peptide 1 (GLP-1) is a peptide hormone encoded by human glucagon gene and secreted by intestinal L cells, which has the following physiological effects: promoting the transcription of insulin gene and increasing the biosynthesis and secretion of insulin in pancreatic islet β cells in a glucose-dependent manner, stimulating the proliferation and differentiation of β cells and inhibiting the apoptosis thereof, thereby increasing the number of pancreatic islet β cells, inhibiting the secretion of glucagon, suppressing appetite and ingestion, as well as retarding the gastric contents emptying, etc. All these functions are advantageous in reducing postprandial blood glucose and maintaining blood glucose at a stable level.

GLP-1 analogues are generally polypeptide compounds formed from amino acids linked in a peptide chain, which differ from the molecular structure of natural GLP-1 in one amino acid and one additional 16-carbon palmitoyl fatty acid side chain, and which have 95% homology to natural GLP-1. Moreover, due to the presence of the fatty acid side chain, the molecule of a GLP-1 analogue is not susceptible to degradation by DDP-IV (dipeptidyl peptidase IV) and can bind to an albumin to obtain a higher metabolic stability with t_{1/2} as long as 12-14 h.

GLP-1 analogues, like proteins, have secondary structures. Secondary structures mainly include α-helix, β-sheet and β-turn, wherein the common secondary structures are α-helix and β-sheet. Secondary structure is maintained through a hydrogen bond formed between a carbonyl group and an amide group on the backbone, which is the main force for stabilizing secondary structure.

The various functions of a polypeptide compound are closely related to the particular conformation thereof. The conformation of a polypeptide is the basis of the functional activities thereof, and when the conformation is changed, the functional activities will change accordingly. When a protein is denatured, its conformation is destroyed, causing the loss of functional activities. In an organism or during the production process of a polypeptide, when a substance specifically binds to a site of a polypeptide chain, a change in the conformation of this polypeptide is triggered, leading to changes of the functional activities, which is referred to as an allosteric effect. The allosteric effect is ubiquitous in organisms and is very important for the regulation of substance metabolism and changes in some physiological functions.

During the preparation process of a polypeptide injection, steps such as dissolution under stirring, adjusting with an acid or a base, and filtration may damage the secondary structure of the polypeptide injection, resulting in loss of activity thereof. Currently, most enterprises or laboratories merely perform a quantity control on the primary structure of a preparation without examination on the control of the secondary structure thereof during the practical development and production process, and therefore the evaluation on pharmaceutical activity of the preparation is not very accurate. In addition, there is still no report on the control of secondary structure of a liraglutide preparation.

### SUMMARY

In light of this, the prevent invention provides a pharmaceutical composition and a manufacturing method thereof. In the present invention, identification and structural analysis of compounds are carried out by infrared spectroscopy to examine the secondary structure of samples obtained under different manufacturing process condition parameters so as to determine the optimal manufacturing method.

In order to achieve the above objects of the present invention, the following technical solutions are provided herein.

The present invention provides a pharmaceutical composition comprising liraglutide, and the manufacturing method thereof comprises mixing liraglutide with an adjuvant in a solvent, stirring at 500∼1100 rpm until homogeneous, and adjusting pH to 7.5∼9.5.

In some specific embodiments of the present invention, a step of filtration is further comprised after adjusting the pH to 7.5∼9.5, wherein the filtration is performed under a pressure of 0.05∼0.18 Mpa.

In some specific embodiments of the present invention, the stirring is performed at a speed of 700∼1000 rpm.

In some specific embodiments of the present invention, the stirring is performed at a speed of 800∼900 rpm.

In some specific embodiments of the present invention, the pH is 7.7∼9.2.

In some specific embodiments of the present invention, the pH is 8.0∼9.0.

In some specific embodiments of the present invention, the filtration is performed under a pressure of 0.07∼0.15 Mpa.

In some specific embodiments of the present invention, the filtration is performed under a pressure of 0.1∼0.12 Mpa.

In some specific embodiments of the pharmaceutical composition of the present invention, the adjuvant comprises one or a mixture of two or more of a buffer, a stabilizer, a preservative, or a pH adjusting agent, wherein:

the mass ratio of liraglutide, the buffer, the stabilizer, the preservative, and the pH adjusting agent is 6 : (1.3∼1.5) : (12.5∼16) : (5∼6) : (0.15∼0.32).

In some specific embodiments of the pharmaceutical composition of the present invention, the mass ratio of liraglutide, the buffer, the stabilizer, the preservative, and the pH adjusting agent is 6:1.42:14:5.5:24.

In some specific embodiments of the present invention, the manufacturing method of the pharmaceutical composition comprises:
Step 1: mixing a buffer and a stabilizer with water to obtain a first solution;
Step 2: mixing liraglutide with the first solution and stirring at 500∼1100 rpm until homogenous to obtain a second solution;
Step 3: mixing a preservative with water to prepare a third solution;
Step 4: mixing the second solution and third solution with water and adjusting the pH to 7.5∼9.5 with a pH adjusting agent; and
Step 5: performing filtration by using a 0.2 µm polyethersulfone filtration membrane under a pressure of 0.05∼0.18 MPa.

In some specific embodiments of the pharmaceutical composition of the present invention,
the buffer comprises one or a mixture of two or more of disodium hydrogen phosphate dihydrate, sodium dihydrogen phosphate, dipotassium hydrogen phosphate, potassium dihydrogen phosphate, sodium phosphate, and sodium citrate;
the stabilizer comprises one or a mixture of two or more of propylene glycol, glycerol, mannitol, glycine, and tromethamine;
the preservative comprises one or a mixture of two or more of phenol, benzoic acid, sodium benzoate, sorbic acid, potassium sorbate, and calcium propionate;
the pH adjusting agent is sodium hydroxide; and
the solvent is water.

In some specific embodiments of the present invention, in the manufacturing method of the pharmaceutical composition, water is added in an amount of 60% (v/v) of the formula amount in Step 1 and 20% (v/v) of the formula amount in Step 3; and in Step 4, water is added to reach 90% (v/v) of the final volume.

The present invention also provides a manufacturing method of the pharmaceutical composition, which comprises mixing liraglutide with an adjuvant in a solvent, stirring at 500∼1100 rpm until homogeneous, and adjusting pH to 7.5∼9.5.

In some specific embodiments of the manufacturing method of the present invention, a step of filtration is further comprised after adjusting the pH to 7.5∼9.5, wherein the filtration is performed under a pressure of 0.05∼0.18 Mpa.

In some specific embodiments of the manufacturing method of the present invention, the stirring is performed at a speed of 700∼1000 rpm.

In some specific embodiments of the manufacturing method of the present invention, the stirring is performed at a speed of 800∼900 rpm.

In some specific embodiments of the manufacturing method of the present invention, the pH is 7.7∼9.2.

In some specific embodiments regarding the manufacturing method of the present invention, the pH is 8.0∼9.0.

In some specific embodiments of the manufacturing method of the present invention, the filtration is performed under a pressure of 0.07∼0.15 Mpa.

In some specific embodiments of the manufacturing method of the present invention, the filtration is performed under a pressure of 0.1∼0.12 Mpa.

In some specific embodiments of the manufacturing method of the present invention, the adjuvant comprises one or a mixture of two or more of a buffer, a stabilizer, a preservative, or a pH adjusting agent, wherein:
the mass ratio of the liraglutide, pH adjusting agent, pH adjusting agent, preservative, and pH adjusting agent is 6 : (1.3∼1.5) : (12.5∼16) : (5∼6) : (0.15∼0.32).

In some specific embodiments of the manufacturing method of the pharmaceutical composition of the present invention, the mass ratio of liraglutide, the buffer, the stabilizer, the preservative, and the pH adjusting agent is 6:1.42:14:5.5:24.

In some specific embodiments of the present invention, the manufacturing method comprises the following steps:
Step 1: mixing a buffer and a stabilizer with water to obtain a first solution;
Step 2: mixing liraglutide with the first solution and stirring at 500∼1100 rpm until homogenous to obtain a second solution;
Step 3: mixing a preservative with water to prepare a third solution;
Step 4: mixing the second solution and third solution with water and adjusting the pH to 7.5∼9.5 with a pH adjusting agent; and
Step 5: performing filtration by using a 0.2 µm polyethersulfone filtration membrane under a pressure of 0.05∼0.18 MPa.

In some specific embodiments of the manufacturing method of the present invention,
the buffer comprises one or a mixture of two or more of disodium hydrogen phosphate dihydrate, sodium dihydrogen phosphate, dipotassium hydrogen phosphate, potassium dihydrogen phosphate, sodium phosphate, and sodium citrate;
the stabilizer comprises one or a mixture of two or more of propylene glycol, glycerol, mannitol, glycine, and tromethamine;
the preservative comprises one or a mixture of two or more of phenol, benzoic acid, sodium benzoate, sorbic acid, potassium sorbate, and calcium propionate;
the pH adjusting agent is sodium hydroxide; and
the solvent is water.

In some specific embodiments of the present invention, water is added in an amount of 60% (v/v) of the formula amount in Step 1 and 20% (v/v) of the formula amount in Step 3, and in Step 4, water is added to reach 90% (v/v) of the final volume.

The present invention provides a pharmaceutical composition comprising liraglutide, and the manufacturing method thereof comprises mixing liraglutide with an adjuvant in a solvent, stirring at 500∼1100 rpm until homogeneous, and adjusting pH to 7.5∼9.5. The process parameters can influence the stability of liraglutide with a significant trend of the changes in oligomers, the maximal single impurity, and the total impurities. It can be seen from the spectra of a homemade preparation which is treated as a liquid sample and loaded for ATR (Attenuated Total Reflection) measurement, there is a strong absorption peak of amide band I (at about 1645 nm⁻¹) and the peak shape is substantially consistent, indicating the presence of α-helix structure. In the present invention, the parameter screening during the manufacturing process of a preparation is determined by examining the secondary structure of a polypeptide, significantly (P < 0.05) improving the stability of a polypeptide medicament and maintaining the pharmaceutical activity thereof.

### BRIEF DESCRIPTION OF THE DRAWINGS

In order to illustrate the technical solutions of the inventive examples or in the prior art more clearly, the accompanying drawings to be used in describing the examples and prior art will be briefly introduced hererinafter.
Figure 1 shows the infrared spectrum of the pharmaceutical composition prepared in Example 1;
Figure 2 shows the infrared spectrum of the pharmaceutical composition prepared in Example 2;
Figure 3 shows the infrared spectrum of the pharmaceutical composition prepared in Example 3;
Figure 4 shows the infrared spectrum of the pharmaceutical composition prepared in Example 4;
Figure 5 shows the infrared spectrum of the pharmaceutical composition prepared in Example 5;
Figure 6 shows the infrared spectrum of the pharmaceutical composition prepared in Example 6;
Figure 7 shows the infrared spectrum of the pharmaceutical composition prepared in Example 7;
Figure 8 shows the infrared spectrum of the pharmaceutical composition prepared in Example 8;

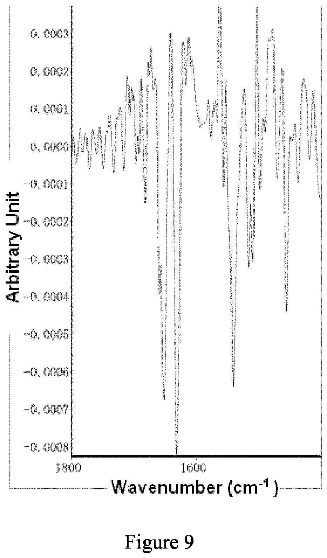

Figure 9 shows the infrared spectrum of the pharmaceutical composition prepared in Comparative Example 1;

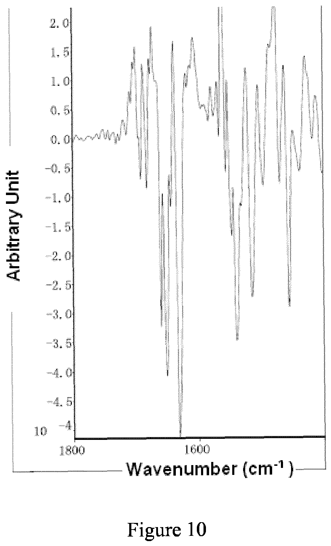

Figure 10 shows the infrared spectrum of the pharmaceutical composition prepared in Comparative Example 2;

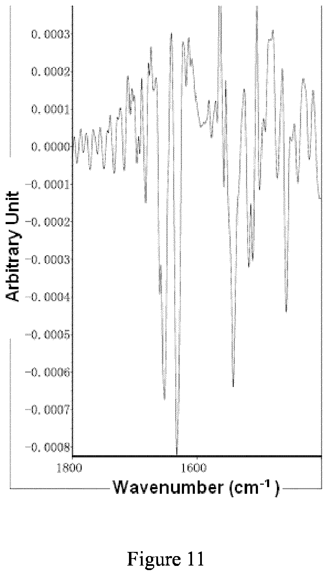

Figure 11 shows the infrared spectrum of the pharmaceutical composition prepared in Comparative Example 3; and

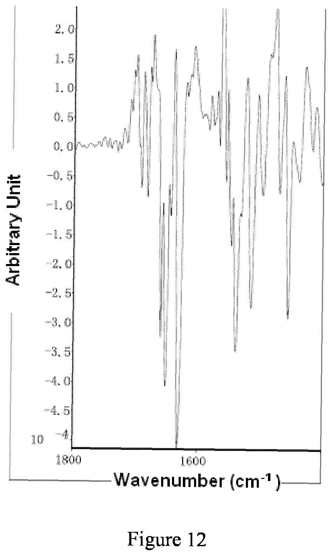

Figure 12 shows the infrared spectrum of the pharmaceutical composition prepared in Comparative Example 4.

### DETAILED DESCRIPTION OF EMBODIMENTS

The present invention discloses a pharmaceutical composition and a manufacturing method thereof, which can be achieved by those skilled in the art through appropriately improving the process parameters in light of the present disclosure. It is particularly pointed out that all similar replacements and modifications are obvious for those skilled in the art and regarded as being included in the present invention. The methods and applications of the present invention have been described by preferred examples, and it is obvious that those skilled in the art can achieve and apply the inventive technique by modifying or appropriately changing and combining the methods and applications described herein without departing from the contents, spirit, and scope of the present invention.

The technical solutions provided by the present invention are:
Stirring speed: 500∼1100 rpm, preferably 700∼1000 rpm, more preferably 800∼900 rpm;
pH range: 7.5∼9.5, preferably 7.7∼9.2, more preferably 8.0-9.0;
Filtration pressure: 0.05∼0.18 MPa, preferably 0.07∼0.15 MPa, more preferably 0.1∼0.12 MPa.

Formulation ratio:
Liraglutide : disodium hydrogen phosphate dihydrate : propylene glycol : phenol : sodium hydroxide = 6 : 1.3∼1.5 : 12.5∼16 : 5∼6 : 0.15∼0.32.

Preferably, substitutes for the adjuvants provided by the present invention are:
disodium hydrogen phosphate dihydrate: sodium dihydrogen phosphate, dipotassium hydrogen phosphate, potassium dihydrogen phosphate, sodium phosphate, and sodium citrate;
propylene glycol: glycerol, mannitol, glycine, and tromethamine;
phenol: benzoic acid, sodium benzoate, sorbic acid, potassium sorbate, and calcium propionate.

Preferably, water is added in an amount of 60% (v/v) of the formula amount in Step 1 and 20% (v/v) of the formula amount in Step 3, and water is added to reach 90% (v/v) of the final volume in Step 4.

The infrared spectra are detected under the following conditions:
Instrument: NICOLET IS10-type FT-IR (Thermo)
Instrument parameters: scanning range, 4000∼650 nm⁻¹ ; resolution, 4 nm⁻¹;
Sample-loading mode: ATR
Method: one drop (about 5 µl) of a liraglutide injection solution sample is taken and dropped on ATR; measurement is performed after the liquid is volatilized; number of scanning: 32 times.

The present invention provides a pharmaceutical composition comprising liraglutide, and the manufacturing method thereof comprises mixing liraglutide with an adjuvant in a solvent, stirring at 500∼1100 rpm until homogeneous, and adjusting pH to 7.5∼9.5. The process parameters can influence the stability of liraglutide with a significant trend of the changes in oligomers, the maximal single impurity, and the total impurities. It can be seen from the spectra of a homemade preparation which is treated as a liquid sample and loaded for ATR (Attenuated Total Reflection) measurement, there is a strong absorption peak of amide band I (at about 1645 nm⁻¹) and the peak shape is substantially consistent, indicating the presence of α-helix structure, which demonstrates that liraglutide has the same secondary structure as that exhibited thereby in a solution. In the present invention, the parameter screening during the manufacturing process of a preparation is determined by examining the secondary structure of a polypeptide, significantly (P < 0.05) improving the stability of a polypeptide medicament and maintaining the pharmaceutical activity thereof.

All the raw materials and reagents used in the pharmaceutical composition and the manufacturing method thereof provided in the present invention are commercially available.

Hereinafter, the present invention will be further explained in combination with examples.

### Example 1

| | |
|---|---|
| Liraglutide | 600 mg |
| Disodium hydrogen phosphate dihydrate | 130 mg |
| Propylene glycol | 1250 mg |
| Phenol | 500 mg |
| Sodium hydroxide | 15 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 500∼1100 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 7.5∼9.5 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 2

| | |
|---|---|
| Liraglutide | 600 mg |
| Disodium hydrogen phosphate dihydrate | 150 mg |
| Propylene glycol | 1600 mg |
| Phenol | 600 mg |
| Sodium hydroxide | 32 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 700∼1000 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 7.5∼9.5 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 3

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 7.5∼9.5 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 4

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 7.7∼9.2 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 5

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 6

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.05∼0.18 MPa to obtain a sample solution.

### Example 7

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.07∼0.15 MPa to obtain a sample solution.

### Example 8

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.1∼0.12 MPa to obtain a sample solution.

### Comparative Example 1

| | |
|---|---|
| Liraglutide | 600 mg |
| Disodium hydrogen phosphate dihydrate | 113 mg |
| Propylene glycol | 1400 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.1∼0.12 MPa to obtain a sample solution.

### Comparative Example 2

| | |
|---|---|
| Liraglutide | 600 mg |
| Disodium hydrogen phosphate dihydrate | 142 mg |
| Propylene glycol | 1740 mg |
| Phenol | 550 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.1∼0.12 MPa to obtain a sample solution.

### Comparative Example 3

| | |
|---|---|
| Liraglutide | 600 mg |
| Disodium hydrogen phosphate dihydrate | 142 mg |
| Propylene glycol | 1400 mg |
| Phenol | 420 mg |
| Sodium hydroxide | 24 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 8.0∼9.0 with a sodium hydroxide solution (0.5 g → 25 ml), and then water was added to reach the final volume.
5) Filtration was performed by using 0.2 µm polyethersulfone filtration membrane under a pressure of 0.1∼0.12 MPa to obtain a sample solution.

### Comparative Example 4

| | |
|---|---|
| Liraglutide | 600 mg |
| Disodium hydrogen phosphate dihydrate | 142 mg |
| Propylene glycol | 1400 mg |
| Phenol | 550 mg |
| Sodium hydroxide | 11 mg |
| Water | to 100 ml |

### Process description:

1) Disodium hydrogen phosphate and propylene glycol were weighed according to the formula amounts respectively and put into a clean beaker, and water was added in 60% of the formula amount; the resultant mixture was stirred until completely dissolved to obtain an adjuvant solution A.
2) The formula amount of liraglutide was weighed and put into a clean beaker, and the adjuvant solution A was added; the resultant mixture was stirred at 800∼900 rpm until homogeneous to obtain a solution 1.
3) Phenol was weighed according to the formula amount, and water was added in 20% of the formula amount; the resultant mixture was stirred until completely dissolved to obtain a solution 2.
4) The solutions 1 and 2 were combined and stirred until homogeneous, and then water was added to reach 90 % of the final volume; pH was adjusted to 6.0∼7.4 with a sodium hydroxide solution (0.5 g → 25 ml), and then water was added to reach the final volume.
5) Filtration was performed by using 0.2 µm polyethersulfone filtration membrane under a pressure of 0.1∼0.12 MPa to obtain a sample solution.

Comparison of the relevant substances is shown in Table 1.

**Table 1. Results of comparison of the relevant substances**

| Example | Oligomers (%) | Maximal Single Impurity (%) | Total Impurities (%) |
|---|---|---|---|
| Example 1 | 0.64 | 0.46 | 3.26 |
| Example 2 | 0.61 | 0.43 | 3.15 |
| Example 3 | 0.42 | 0.38 | 2.41 |
| Example 4 | 0.43 | 0.34 | 2.29 |
| Example 5 | 0.32 | 0.24 | 2.09 |
| Example 6 | 0.32 | 0.23 | 1.35 |
| Example 7 | 0.22 | 0.14 | 1.20 |
| Example 8 | 0.19 | 0.08 | 1.19 |
| Comparative Example 1 | 1.24 | 0.55 | 3.64 |
| Comparative Example 2 | 0.72 | 1.70 | 5.20 |
| Comparative Example 3 | 1.12 | 1.05 | 6.42 |
| Comparative Example 4 | 0.55 | 0.84 | 4.75 |

It can be seen from the above data that, in examples 1-8, the process parameters influence the stability of liraglutide, and the changes of oligomers, the maximal single impurity, and the total impurities contents are significant (P < 0.05).

The oligomers, the maximal single impurity, and the total impurities contents in comparative examples 1-4 which are beyond the protection scope are significantly influenced.

### 3.2 Comparison of the infrared spectra is shown in Table 2.

**Table 2. Results of infrared spectra comparison**

| Examples | Absorption wavelength of amide band I (nm⁻¹) |
|---|---|
| Example 1 | 1654 |
| Example 2 | 1652 |
| Example 3 | 1641 |
| Example 4 | 1640 |
| Example 5 | 1642 |
| Example 6 | 1642 |
| Example 7 | 1644 |
| Example 8 | 1645 |
| Comparative example 1 | 1636 |
| Comparative example 2 | 1655 |
| Comparative example 3 | 1632 |
| Comparative example 4 | 1652 |

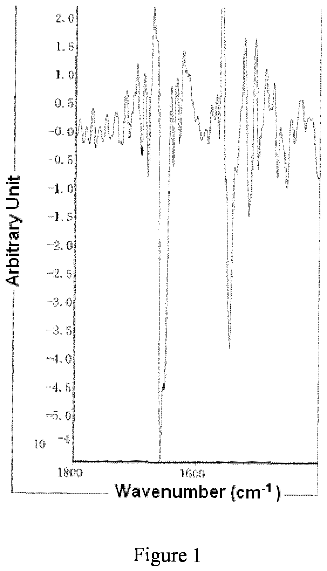

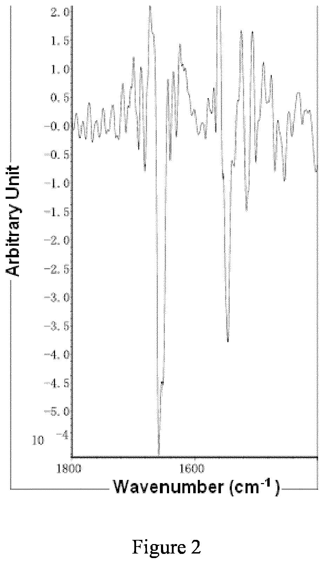

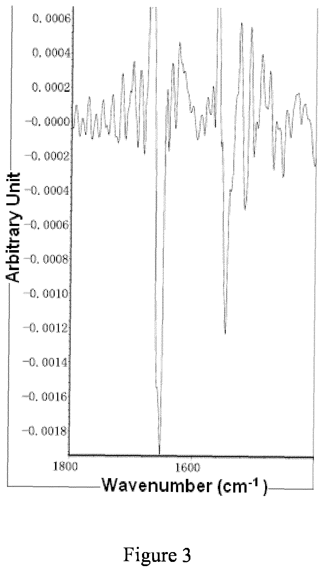

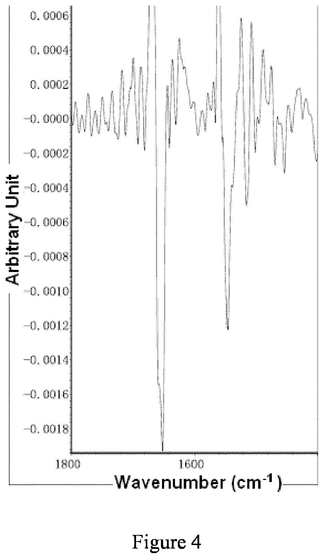

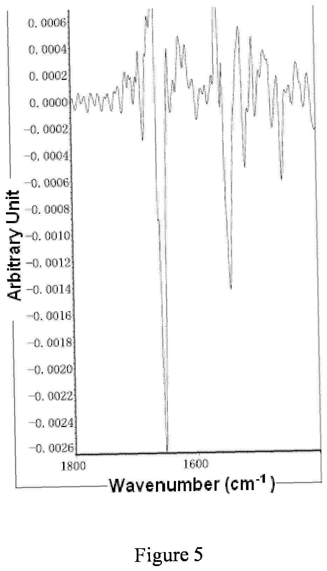

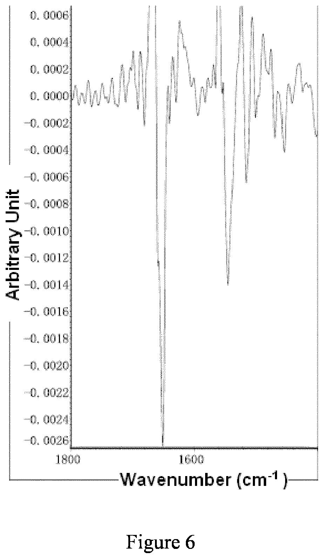

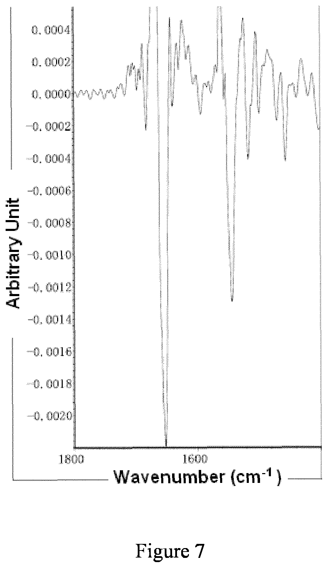

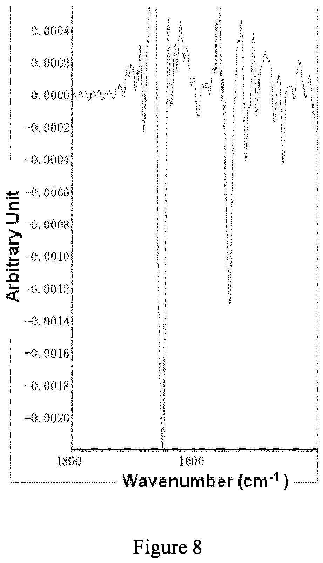

It can be seen from the spectra (Figures 1-8) of treated liquid samples of homemade preparations determined by ATR (Attenuated Total Reflection), there is a strong absorption peak of amide band I (at about 1645 nm⁻¹) and the peak shape is substantially consistent, indicating the presence of α-helix structure, which demonstrates that liraglutide has the same secondary structure as that exhibited thereby in a solution.

It can be seen from the above data, different formulating processes significantly influence the maximal absorption position of amide band I of liraglutide (above ±2 nm) and influence the maximal absorption peak shape to some extent thereof.

It is demonstrated that controlling the parameter screening during the manufacturing process of a preparation by examining the secondary structure of a polypeptide significantly (P < 0.05) improves the stability of a polypeptide preparation and maintaining the pharmaceutical activity thereof.

Those mentioned above are merely preferred embodiments of the present invention, and it should be noted that one of ordinary skill in the art can further make several improvements and modifications without departing from the principle of the present invention, and these improvements and modifications should be also regarded as within the protection scope of the present invention.

The pharmaceutical composition and the manufacturing method thereof provided by the present invention are introduced in detail above. Herein, specific examples are presented to explain the principle and embodiments of the present invention, and the above description of the examples are provided only to help understanding the method and the central idea of the present invention. It should be noted that those skilled in the art can further make several improvements and modifications to the present invention without departing from the principle of the present invention, and these improvements and modifications also fall into the protection scope of the claims of the present invention.

## Claims

1. A pharmaceutical composition comprising liraglutide, wherein manufacturing method thereof comprises mixing, liraglutide with an adjuvant in a solvent, stirring at 500∼1100 rpm until homogeneous, and adjusting pH to 7.5∼9.5.

2. The pharmaceutical composition according to claim 1, wherein a step of filtration is further comprised after adjusting the pH to 7.5∼9.5, wherein the filtration is performed under a pressure of 0.05∼0.18 Mpa.

3. The pharmaceutical composition according to claim 1 or 2, wherein the adjuvant comprises one or a mixture of two or more of a buffer, a stabilizer, a preservative, and a pH adjusting agent, wherein:
the mass ratio of liraglutide, the buffer, the stabilizer, the preservative, and the pH adjusting agent is 6 : (1.3∼1.5) : (12.5∼16) : (5∼6) : (0.15∼0.32).

4. The pharmaceutical composition according to claim 3, wherein the manufacturing method thereof comprises:
Step 1: mixing the buffer and the stabilizer with water to obtain a first solution;
Step 2: mixing liraglutide with the first solution and stirring at 500∼1100 rpm until homogenous to obtain a second solution;
Step 3: mixing a preservative with water to prepare a third solution;
Step 4: mixing the second solution and the third solution with water and adjusting the pH to 7.5∼9.5 with the pH adjusting agent; and
Step 5: performing filtration by using a 0.2 µm polyethersulfone filtration membrane under a pressure of 0.05∼0.18 MPa.

5. The pharmaceutical composition according to any one of claims 1 to 4, wherein:
the buffer comprises one or a mixture of two or more of disodium hydrogen phosphate dihydrate, sodium dihydrogen phosphate, dipotassium hydrogen phosphate, potassium dihydrogen phosphate, sodium phosphate, and sodium citrate;
the stabilizer comprises one or a mixture of two or more of propylene glycol, glycerol, mannitol, glycine, and tromethamine;
the preservative comprises one or a mixture of two or more of phenol, benzoic acid, sodium benzoate, sorbic acid, potassium sorbate, and calcium propionate;
the pH adjusting agent is sodium hydroxide; and
the solvent is water.

6. A method of manufacturing a pharmaceutical composition, comprising mixing liraglutide with an adjuvant in a solvent, stirring at 500∼1100 rpm until homogeneous, and adjusting pH to 7.5∼9.5.

7. The method according to claim 6, wherein a step of filtration is further comprised after adjusting the pH to 7.5∼9.5, wherein the filtration is performed under a pressure of 0.05∼0.18 Mpa.

8. The method according to claim 6 or 7, wherein the adjuvant comprises one or a mixture of two or more of a buffer, a stabilizer, a preservative, and a pH adjusting agent, wherein:
the mass ratio of liraglutide, the buffer, the stabilizer, the preservative, and the pH adjusting agent is 6 : (1.3∼1.5) : (12.5∼16) : (5∼6) : (0.15∼0.32).

9. The method according to claim 8, comprising the following steps:
Step 1: mixing the buffer and the stabilizer with water to obtain a first solution;
Step 2: mixing liraglutide with the first solution and stirring at 500∼1100 rpm until homogenous to obtain a second solution;
Step 3: mixing a preservative with water to prepare a third solution;
Step 4: mixing the second solution and third solution with water and adjusting the pH to 7.5∼9.5 with a pH adjusting agent; and
Step 5: performing filtration by using a 0.2 µm polyethersulfone filtration membrane under a pressure of 0.05∼0.18 MPa.

10. The method according to any one of claims 6 to 9, wherein:
the buffer comprises one or a mixture of two or more of disodium hydrogen phosphate dihydrate, sodium dihydrogen phosphate, dipotassium hydrogen phosphate, potassium dihydrogen phosphate, sodium phosphate, and sodium citrate;
the stabilizer comprises one or a mixture of two or more of propylene glycol, glycerol, mannitol, glycine, and tromethamine;
the preservative comprises one or a mixture of two or more of phenol, benzoic acid, sodium benzoate, sorbic acid, potassium sorbate, and calcium propionate;
the pH adjusting agent is sodium hydroxide; and
the solvent is water.
